# EUROPEAN PATENT APPLICATION

(11) **EP 4 298 990 A1**
(43) Date of publication of application: **03.01.2024**
(21) Application number: 22382621.5
(22) Date of filing: 30.06.2022
(51) Int. Cl.: A61B 5/00, A61B 5/11, A61B 5/389

(54) **WEARABLE APPARATUS TO MONITOR A MUSCULOSKELETAL CONDITION OF A JOINT, LIMB AND MUSCLES AND ALSO TO STIMULATE THEREOF, AND RELATED SYSTEMS**

(71) Applicant: Imaz Technology Innovation, S.L., 08019 Barcelona (ES)
(72) Inventor: Ahmadi Zeidabadi, Maziar, Barcelona (ES); Florentina Sirbu, Georgiana, Barcelona (ES); Martínez García, Herminio, Barcelona (ES); Rodríguez Martínez, Gerard, Barcelona (ES)
(74) Representative: Isern Patentes y Marcas S.L.

(57) **Abstract**

The present invention discloses a wearable apparatus and related systems to monitor a musculoskeletal condition of a joint, limb and muscles and also to stimulate thereof, comprising a wearable structure configured to fit on the joint, limb, or the skin adjacent to the muscle, sensing means attachable to the wearable structure configured to detect and measure activity parameters of said joint, limb or muscles, and to generate sensing data signals related with the musculoskeletal condition of the joint or limb and/or related with the activity parameters measured, stimulating means attachable to the wearable structure configured to provide a stimulation action to the joint, limb or muscles; and control means data linked with the sensing means and with the stimulating means, configured to monitor the musculoskeletal condition and the activity of the joint, limb or muscles and further configured to manage the stimulating means.

## Description

### TECHNICAL FIELD OF THE INVENTION

The present invention is related to apparatus, devices, systems and/or methods for monitoring the musculoskeletal conditions and biomechanical behaviours of the joints, limbs and muscles. Furthermore, the present invention foresees identifying a musculoskeletal current status of the joints, limbs and muscles for, for example, diagnostic purposes, and also allow the stimulation of said joints, limbs and muscles for, for example, rehabilitation purposes, among others.

### STATE OF THE ART

In medicine or healthcare, wearable technologies are defined as autonomous, non-invasive devices that capture, analyse, and aggregate physiological data to improve personal healthcare and well-being. Wearable technology has been used to date almost exclusively for physical enhancement purposes, boosted by the growing consumer demand to monitor their own health.

The integration of the wearable technology with augmented reality (AR), Big Data, artificial intelligence (Al) and cloud computing solutions, as well as falling prices for sensors, open-source application programming interfaces (APIs), frameworks and libraries, is enabling faster and more cost-effective solutions within the Internet of Things (loT) ecosystem.

Recent advances are adding value to healthcare with a focus on diagnosis, monitoring, treatment and injure prevention. These benefits are seen throughout the healthcare value chain with benefits that include personalization, early diagnosis, remote patient monitoring (RPM), medication adherence, information libraries, and better decision-making management, while reducing healthcare costs. In addition, the increasing demand and functionality has attracted the attention of insurers and companies in the provision of wearable health technology to consumers and employees for its extensive benefits.

In particular, the wearable technologies have been focused in wearable apparatus like "Smart-gloves" or "Smart knee braces" or simply "Smart braces" which, through different sensors, could be employed to collect representative measurements of the experienced along a user's limb when a movement or force is applied or received thereon.

Wearable apparatus could be further provided with inertial measurement sensors, particularly multi-axis accelerometers, which are intended to detect the position or orientation of the limb, among others.

Furthermore, in a wearable apparatus, other sensors like flex sensors are known and arranged on the fingers of the user, wherein, due to those flex sensors, it is possible to detect specific operations related with the movement of the user's fingers, such as the release an object.

On the other hand, in addition to the detection or measurements of the experienced along a user's limb when a movement or force is applied or received thereon, other documents of the state of the art disclosing further non-invasive devices for assisting in the treatment of any kind of joints or limb disorders, comprising but not exclusive of those of joint, limb, and spine disorders, including a brace, sleeve, flexible pad, or any combination thereof, with a plurality of electrodes disposed thereon, and the electrodes configured to transmit at least three electrophysical modalities and through a stimulation control unit provided with an interactive software to establish a controlled sequence of transmission of the three electrophysical modalities and communicating the controlled sequence to the electrodes to stimulate the limb.

Despite the aforementioned wearable apparatus are intended to detect, measure or asses many operations or movements of the limbs or even to stimulate the limb for the treatment of a disorder condition, neither of them is designed to comprehensive reduce, predict or recover from possible injuries coming from unhealthy movements of the limbs or the joints, and nor to further enable the user to feeling physical sensations that take place under a virtual reality experience.

Therefore, improvements in this regard are needed.

### DESCRIPTION

The above objects and others are achieved by means of a wearable apparatus to monitor a musculoskeletal condition of a joint or limb and also to stimulate thereof of a user as set forth in the independent claim 1.

The claims dependent on claim 1 describe other characteristics of the present invention or variants to the main new and inventive wearable apparatus.

The wearable apparatus comprises a wearable structure, adapted to be comfortable worn in the respective part of the body, i.e., joint or limb, wherein said wearable structure is configured to attach on it a different sensor arrays and/or means for stimulation that will be described later on.

Within the scope of the invention, it must be understood that, apart from the joint or limb, the wearable structure is intended to be worn in those parts of the body where the monitoring of the musculoskeletal condition is needed and/or the physical stimulation must be performed. For example, the wearable structure could be a brace or cuff, or sticking patch or plaster, or similar.

Also, within the scope of the invention, musculoskeletal is a term referred to the system of a human body that include bones, cartilage, ligaments, tendons and connective tissues. In this regard, the skeleton provides a framework for the muscles and other soft tissues. Together, they support the body's weight, maintain the posture and help moving. According to this "monitoring a musculoskeletal condition" refers to detect or monitor the status of the include bones, cartilage, ligaments, tendons and connective tissues of the limb or joint where the wearable apparatus is worn.

As for measure or detect a musculoskeletal condition of the joint and/or the limb, the apparatus comprises sensing means attachable to the wearable structure, wherein the sensing means are further configured to measure activity parameters e.g., the movements and/or forces exerted, of said joint and/or limb and/or muscles, and to generate sensing data signals related with the musculoskeletal condition and/or the activity parameters measured. The sensing means comprises a suitable sensor or a plurality of sensors, depending on the condition or parameter to be measured or detect, which make it possible to measure, among others, the movement or orientation of the joint and/or the limb, a gripping force, for example, of the hand, or detecting bio-marks of the joint and/or the limb, for example, an EMG sensor disposed on the wearable structure configured to monitor the muscular activities of the joint and/or the limb.

The sensor or the plurality of sensors are disposed in a releasable arrangement to the wearable structure as for making a modular apparatus, provided with the suitable sensors attached on it for specific detections purposes. Also, the plurality of sensors can be configured as module of sensors, wherein any sensor of the referred module is releasably coupled within the module, and can be operatively connected (data signal connection) between them and with the control means. Furthermore, the module could be provided with a controller (its own control means) in order to manage the sensors and to transmit/receive data signals to/from the control means. Several modules of sensors can be attachable along the wearable structure.

Further, the apparatus comprises stimulating means attachable to the wearable structure configured to provide a stimulation action to the joint and/or the limb and/or the muscles; wherein the stimulation action could be a temperature change, e.g., heating the joint and/or the limb, and/or vibration, e.g., hammering or drumming on the skin over the joint or limb, and/or electrostimulation, e.g., electrodes that causes electrical pulses on the joint, limb or even directly on the muscles.

The stimulating means comprises a stimulating element or a plurality of stimulating elements releasable arranged on the wearable structure. In an analogue way to the sensor module, stimulating elements could be disposed in a stacked manner as for forming a stimulating module, wherein any stimulating element of said module is releasably coupled within the module, and can be operatively connected (data signal connection) between them and with the control means. Furthermore, the stimulating module could be provided with a controller (its own control means) in order to manage the stimulating elements and to transmit/receive data signals to/from the control means. Several stimulating modules can be attachable along the wearable structure.

Furthermore, the sensing means and the stimulating means can be arranged in a module, namely a compact module, that may be disposed along the wearable structure where the sensing data and or the stimulations actions must be performed.

Hence, the wearable apparatus of the invention has been drawn up as a modular apparatus, wherein the sensors or stimulating elements are chosen and arranged according to specific requirements of a treatment, detections or stimulating actions to be performed.

Moreover, the apparatus comprises control means in data signal connection with the sensing means and with the stimulating means, wherein the control means are configured to monitor the musculoskeletal condition and the activity of the joint and/or limb and/or muscles based on the sensing data signals; and are further configured to manage the stimulating means based on the sensing data signal and/or on external data signals.

Within the scope of the invention "control means" must be understood as physical mean or virtual mean capable of the reception of data, calculations based on those data, and determine data/functioning instructions based on the calculations, that it will be used later. The control means are provided with a suitable computer software program that make it possible to perform the functions of the present invention.

According to the above, the sensing means detect a status or a musculoskeletal condition of the joint and/or limb and the control means are inputted with the sensing data generated from the detection of the status or condition. From here, the control means can manage the stimulating means for causing the stimulation actions, and/or perform a comparison between the sensing inputted data signals and external data signals, taking the stimulation actions based on the result of the comparison. In this regard, external data signals include, but are not limited to, those data signals that coming from, for example, a pre-established database or a real time data coming from, for example, another same or similar wearable apparatus disposed on a joint and/or limb, and/or data from sensors arranged on another joint and/or limb, and/or from the actions performed under a virtual reality experience.

In the case of the pre-established database, this database could store data from previous measurements or detections by the sensing means, e.g., from the user (apparatus historical data usage of the user) to assess the evolution of a treatment, during a recovery treatment. Also, the database can contain data from different users or other types of stored data that could be suitable comparable with the sensing data from the sensing means.

Regarding the real time data, that type of data is obtained from the measurements or detections of other sensors (sensing means) rather than the detection of the sensing means of the wearable device. For example, a first wearable device of the invention is worn in the left hand of the user, and a second wearable device of the invention is worn in the right hand of the user. Thus, the sensing data signals from the first device inputting the control means of the second device, and control means of the latter performing the comparison. This feature would be useful in order to assess the musculoskeletal condition of the limbs, e.g., the hands in this case, setting a comparison between them. Moreover, when either of the hands is in a recovery treatment from an injuring condition or treating a musculoskeletal disorder condition, the sensing data from the healthy hand can be compared to those sensing data of the injured hand, and the result of this comparison make it possible to the control means of the device worn on the injured hand to activate the stimulating means that are useful for recover o treat said injured hand. This example can be applied in other joints or limbs of the body, mutatis mutandis. Further, either of the first or second device can be worn in a different user.

Also, as it was stated, the external data signals could come from actions under a virtual reality experience. In this case, for example, if, in a virtual reality or simulation, the user holds a warm object with the right hand, the control means of the wearable device worn in the right hand of the user are inputted with sensing data relative to the gripping and/or the warm feeling, and/or the texture of a surface feeling, and through a comparison with the actual sensing data, activate the stimulating means for the user to feel in the hand the gripping and/or the warming sensation, and/or the texture sensation, for example, activating a heater or a vibrator disposed in the wearable structure,.

On the other hand, implemented with the control means there is an artificial intelligence (IA hereinafter) configured to generate action protocols for the prevention and/or recovery of a specific musculoskeletal of the joint or limb. It is important to highlight that the aforementioned action protocols are generated from a training of the IA based on the monitoring of the movements, applying forces and muscle strength of the hand and/or the wrist and all the data signals inputting from the different sensors of the apparatus. Alternatively, machine learning or deep learning could be further included within the control means for the generation of action protocols for the prevention and/or recovery of a specific musculoskeletal disorder condition of the joint or limb.

The wearable apparatus of the present invention makes it possible performing an accurate diagnosis of a musculoskeletal condition of the joint and/or the limb, allowing, among others, advantageously reducing possible damage from a wear and tear of the joints and also the limbs, avoiding or correcting unhealthy movements of limbs, preventing injuries, and, creating awareness in the users about the right movements or positions of the joints and/or limbs. Furthermore, and based on the diagnosis of the musculoskeletal condition of the joint and/or the limb, the innovative wearable apparatus allows a comprehensive recovering from an injure or a musculoskeletal disorder condition, through a stimulation action by the stimulating means on the joint or limb carried out by the closed loop control by the control means feed with the sensing data.

Alternatively, within the sensing means there is an automatic gain control (AGC from now on) configured to, based on the sensors data signals, calibrate, by hardware, the sensors of the sensing means. The AGC allowing mechanical, or analogical sensors, or resistive sensors, such as flex sensors or press sensors, to be calibrated directly by hardware from the data signals of said mechanical sensors inputted in the control means, and also can takes into account the data signals from inertial sensors, e.g., accelerometers or gyroscopes.

Alternatively, the calibration of the sensors could be performed by "software" by the control means under a comparison between a pre-set measure of those sensors and a further measure, e.g., in operation.

On the other hand, the wearable apparatus comprises displaying means operatively connected with the sensing means and/or with the stimulating means and/or with the controller, being configured said displaying means to visual feedback of the musculoskeletal condition and/or the activity of the joint and/or limb and/or muscles.

Alternatively, the control means are configured to transmit data and/or receive data to/from a portable electronic device, wherein the portable electronic device is configured to display data of the sensing means and/or the stimulating means. Through the portable electronic device is possible real-time monitoring of the joint and/or limb with accurate and instantaneous detection of the musculoskeletal condition and/or biofeedback of the joint, limb or muscle to assist the control means and the user to identify "what" movement, "when" and "how" occurs, in order to correct or reduce stress and tension in the joint or limb, avoiding possible damage.

On the other hand, the wearable structure is made of a suitable ergonomic design and materials that make it adaptable said wearable structure to different range of sizes and shape of the body where it will be worn, and also that make it possible to attach any sensor/transducer of the sensing means and/or any element (e.g., an electrode, heater or similar) being part of the stimulating means. In an exemplary embodiment the structure is formed through an in-mold electronic technique that allows integration of electronic components within biocompatible polymers that provides an advantageously ergonomic design and modularity make it possible to releasable attach the components.

As it was stated in the previous paragraphs, the control means can be loaded/fed/inputted by the sensing data signals or stimulation data signals from an analogue wearable device or other sensors, the present invention further discloses a system to monitor a musculoskeletal condition of a joint, limb and/or muscles and also to stimulate thereof, the system comprises at least two wearable apparatus of the invention as it has been defined above, being placed each of those wearable apparatus in different joint, limb and/or muscles, wherein the control means of each wearable apparatus are operatively connected between them in order to share musculoskeletal conditions and/or sensing data signals and/or stimulation data signals or actions.

The control means of each wearable apparatus are preferably wirelessly network connected, hence the sensing data signals and/or stimulation data signals can be transmitted with compatible communication technology such as Bluetooth^{®}, Bluetooth mesh, among other Wi-Fi technologies, and any other wireless communication technology.

Other advantages and features of the present invention will become clearer upon reading of the following non-restrictive description of preferred embodiments thereof, given as an example only with reference to the accompanying drawings.

### BRIEF DESCRIPTION OF THE FIGURES

The foregoing and other advantages and features will be better understood from the following detailed description of exemplary embodiments with reference to the accompanying drawings, which should be considered by way of illustration and not limitation, wherein:
- Fig. 1 is a view of a general embodiment of the wearable device of the invention.
- Fig. 2 is a view of a first preferred embodiment of the wearable device of the invention, wherein the wearable structure is configured to be worn in the hand and in the forearm of the user.
- Fig. 3 is a detailed view of the first preferred embodiment of the wearable device of the invention, wherein is shown modules of sensors and stimulating modules are disposed in the index finger.
- Fig. 4 is a view of a second preferred embodiment of the wearable device of the invention, wherein the wearable structure is configured to be worn in the lower limb about the knee of the user.

### DETAILED DESCRIPTION OF AN EXEMPLARY EMBODIMENT

In the following detailed description, specific details are set forth in the form of examples to provide a thorough understanding of the relevant teachings. However, it will be apparent to those skilled in the art that the present teachings can be implemented without such details.

### General embodiment

The present invention is directed to a wearable apparatus 1, apparatus 1 hereinafter, to monitor a musculoskeletal condition of a joint, limb and muscles and also to stimulate thereof.

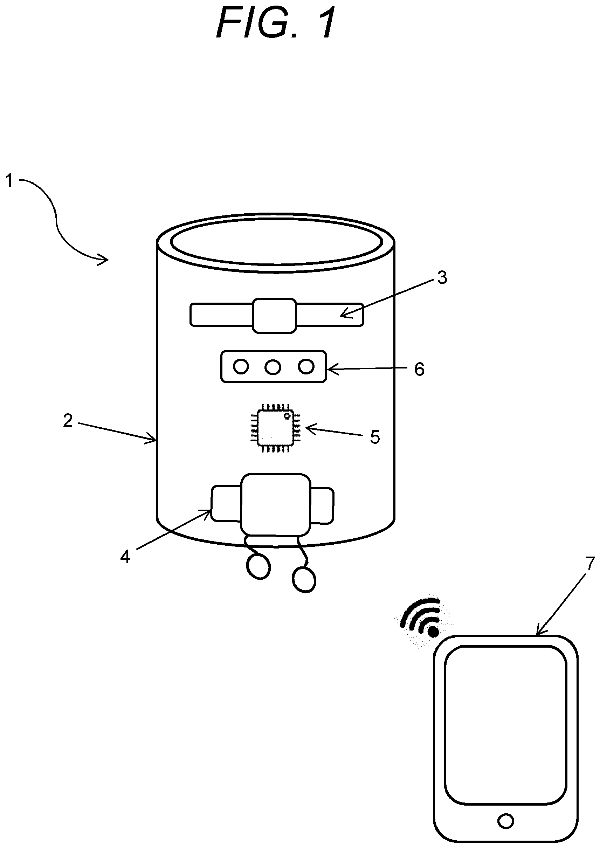

As can be seen in the figure 1, the apparatus 1 comprising a wearable structure 2 configured to fit over the user's joint and/or the limb, and/or adjacent to the skin of the muscle.

Sensing means 3 are attachable to the wearable structure 1, being configured said sensing means 3 to detect the musculoskeletal condition of the joint and/or limb and/or muscles, to measure activity parameters of said joint and/or limb and/or muscles, and to generate sensing data signals related with the musculoskeletal condition and/or the activity parameters measured.

Further, attachable to the wearable structure 2, there are stimulating means 4 attachable to the wearable structure 2, wherein the stimulating means are configured to provide a stimulation action (e.g., carrying out electrical stimulation, heating, vibrating) over the joint and/or the limb and/or the muscles. Also, the stimulating means

Moreover, the apparatus 1 comprises control means 5 in data signal connection with the sensing means 3 and/or with the stimulating means 4, wherein the control means 5 are configured to monitor the musculoskeletal condition and/or the activity of the joint and/or limb and/or muscles based on the sensing data signals from the sensing means 4; and are further configured to manage the stimulating means 4 based on the sensing data signal and/or on external data signals.

An artificial intelligence (IA hereinafter) is implemented with the control means 5. As the control means 5 foresee the reception of the sensing data signals and/or the stimulation data signals, said IA could be trained in order to generate action protocols for the prevention and/or recovery from and injuring and/or a specific musculoskeletal disorder of joint and/or limb and/or muscles. Further the IA could be trained to generate action protocols during a virtual reality experience, i.e., for performing stimulation actions on the stimulating means under previous virtual reality experiences.

In any of the embodiments of the invention, the sensing means 3 comprises at least a sensor or a plurality of sensors, individually disposed and/or stacked manner (module of sensors) releasable arranged on the wearable structure 2, wherein said sensor or sensors could be chosen from, without limit to, pressure sensors, movement sensors (e.g., accelerometers and/or gyroscopes), bio-marks sensors (oxygen and/or EMG sensors), among others. The sensor or sensors are disposed in a releasable coupling to the wearable structure 2 as for making a modular apparatus 1, provided with the suitable sensors attached on it for specific detections purposes. Also, as module of sensors, any sensor of the referred module is releasably couplable within the module, and can be operatively connected (data signal connection) between them and with the control means 5. Several modules of sensors can be attachable along the wearable structure 2.

The sensing means 3 comprises an automatic gain control (AGC) configured to, based on the sensors data signals, calibrate, by hardware, the sensors of the sensing means. The AGC allowing mechanical, analogical or resistive sensors, such as flex sensors or press sensors, to be calibrated directly by hardware from the data signals of said mechanical sensors inputted in the sensing means, and also can take into account the data signals from inertial sensors, e.g., accelerometers or gyroscopes.

Alternatively, the sensor or the plurality of sensors can be calibrated by software. This can be done stablishing a pre-set measure (recording an initial measurement) of the sensor (or sensors) stored by the control means 5, having this pre-set measure as a reference measure, and, comparing said pre-set measure with and measure took by the sensor (or sensors) thereafter. The control means 5 performing a comparison between at least these two measurements and adjust or calibrate the sensor (or sensors) according to the pre-set measure.

In the same way, in any embodiment of the invention, the stimulating means 4 comprises a stimulating element or a plurality of stimulating elements releasable arranged on the wearable structure 2, wherein said stimulating element or elements could be chosen from, without limit to, heaters, vibrators, electrodes, among others. The stimulating element or elements are disposed in a releasable coupling to the wearable structure 2 as for making modular the apparatus 1 of the invention, which is also provided with the suitable stimulating elements attached on it for specific stimulation action purposes. Further, the stimulating elements could be disposed in a stacked manner as for forming a stimulating module, wherein any stimulating element of said module is releasably coupled within the module, and can be operatively connected (data signal connection) between them and with the control means 5. Several stimulating modules can be attachable along the wearable structure.

Hence, the wearable apparatus 1 of the invention has been drawn up as a modular apparatus, wherein the sensors or stimulating elements or modules are chosen and arranged according to specific requirements of a treatment, detections or stimulating actions to be performed, and can be operatively connected between them.

In the preferred embodiment, the sensor, stimulation element and/or modules are network paired trough an innovative Bluetooth^{®} technology, namely Bluetooth Mesh. As the name itself indicates, it is a technology that allows to create a network of devices wirelessly interconnected with each other. This makes it possible to connect a number of devices to a main device (control means 5) because each of the devices is acting as communicator and receiver between them, enabling a whole customization of the wearable apparatus. The small design helps to the portability and modularity of the apparatus 1, being able to connect multiple apparatus in every part of the body of the user to have a better approach of the medical purposes.

Further, the apparatus 1 comprises displaying means 6 operatively connected (in data connection) with the sensing means 3, and/or with the stimulating means 4 and/or with the control means 5, being configured said displaying means 6 to visual feedback of the musculoskeletal condition and/or the activity of the joint and/or limb and/or muscles, and/or a general functioning of the apparatus. In exemplary embodiments, the displaying means 6 are in the form of LEDs for indicate a functioning status of the apparatus 1, e.g., pairing status of the sensors and/or the stimulating elements, state of charge of a battery, when the apparatus is provided with this type of energy supply, etc. Other displaying means as screen, tactile screen and/or acoustic means could be considered with the scope of the invention.

Also, the control means 5 are configured to transmit data and/or receive data to/from a portable electronic device 7, device 7 hereinafter, wherein the device 7 is configured to display data of the sensing means 3 and/or the stimulating means 4. Furthermore, the sensing means 3 and/or the stimulating means 4 could be configured to directly send the sensing data signals and/or the stimulating data signals to the device 7, wherein the device 7 actuate as the control means as it extensively described. In the device 7 there is provided a computer implemented program that controls and enables the whole operation of the apparatus 1 of the invention, and, consequently, of its preferred embodiments.

The apparatus 1 allows sensorial stimulation, through the stimulating means 4, as it is capable of generating biofeedback of the joint or limb, such as temperature, vibration, electrostimulation, etc. As an example, these sensorial stimulations can be used for different applications, such as rehabilitation and recovery, by applying heat during the exercises. That is to say that the apparatus is able either monitoring rehabilitation progress and applying stimulations (heating, vibration, electro-stimulating, etc.).

Another example could be for virtual reality application, the device can create real-time sensation of heat and/or texture in a virtual reality, when the user touches an object in a virtual reality, the device can also stimulate a sensation of heat by a heater and/or texture sensation using piezo electric vibration at different frequency in real-time.

### Exemplary embodiment 1: Upper limb (hand and forearm)

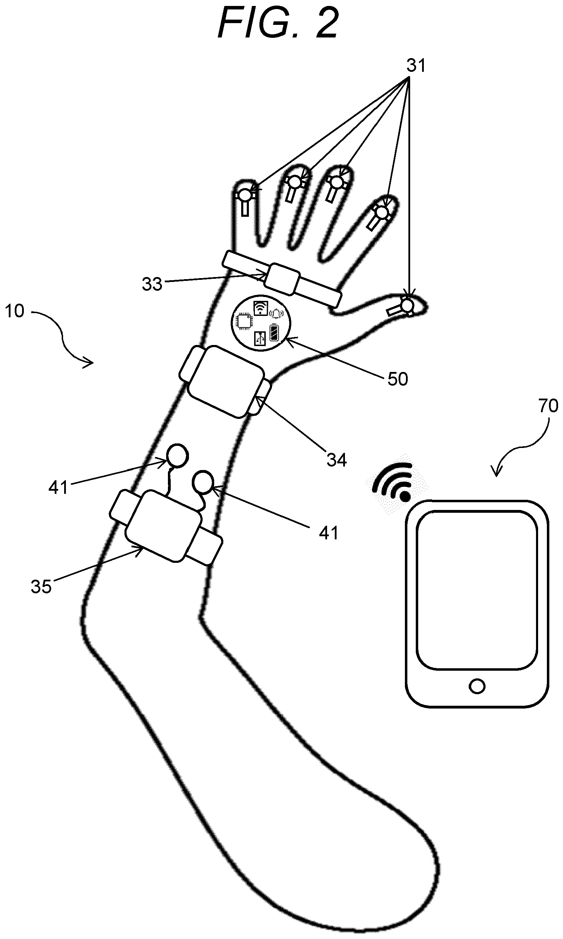

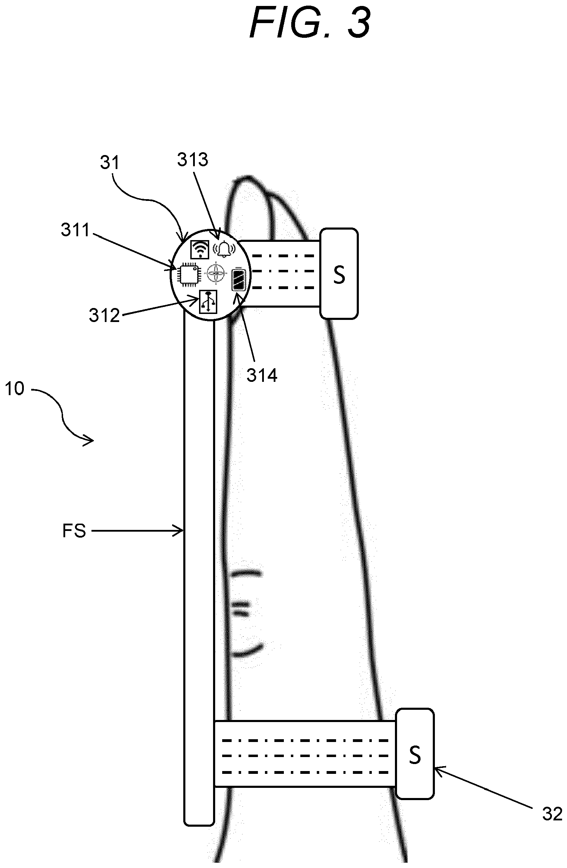

In a first exemplary embodiment, shown in figures 2 and 3, the present invention discloses a wearable apparatus 10, apparatus 10 hereinafter, to monitor a musculoskeletal condition of the joints of the upper limb and the muscles thereof and also to stimulate said joints or muscles, comprising a wearable structure (not shown) in the form of a glove or similar intended to be worn about the hand, with a sleeve extension to also cover at least partially the wrist and the forearm, being said wearable structure configured to integrate electronic components or circuits as well as the different sensors of the sensing means and the stimulating elements of the stimulating means and the control means forming the apparatus 10.

The apparatus 10 comprises, as sensing means 30, a plurality of sensors and/or module of sensors disposed along the upper limb. Specifically, there is a module of sensors 31 at the fingertip of at least one finger and, in the shown embodiment of the figure 2, in the fingertip of every finger of the hand. Furthermore, in the embodiment of the figure 3, there is a module of sensors 32 at the base of at least one finger.

Also, another module of sensors 33 is disposed about the palm portion of the hand, and there is also a module of sensors 34 and 35 disposed at the wrist and at the forehand, respectively.

As it is shown in the figure 3, a flex sensor (FS) or similar is disposed on the wearable structure along at least one finger of the hand, being said flex sensor (FS) also operatively connected with, for example, the module of sensors 31 and/or a stimulation module and/or a compact module.

In the light of the foregoing, the wearable structure (not show in this embodiment) must be configured to enable the releasable attachment of the different sensors and/or the different modules of sensors 31, 32, 33, 34, 35.

As it has been previously explained, each of the module of sensors are configurable in order to attach/detach the required sensor or sensors for a specific detection and/ measurement. For example, the module of sensors 31 can be provided with a pressure sensor, a temperature sensor and an oxygen sensor; the same arrangement of sensor is also possible for the module of sensors 32 with further an orientation and/or movement sensor (accelerometer and/or gyroscope), whilst the module of sensors 34 may be provided with additionally pulse and other bio-marks sensor, and the module of sensors 35 may be provided with also an EMG sensor, among others.

Further, within each module of sensors there will be at least a stimulation element (not shown) of the stimulating means to stimulate a specific part of the upper limb. For example, in the module of sensors 35 at the forearm, electrodes 41 are disposed to cause electrical stimulation of the forearm. In another example, a vibrational element (not shown) is disposed in the module of sensors 31 to provide a vibrational sensation when, for example, a user touch something. In a similar way, each of the module of sensors, are configurable in order to attach/detach the required stimulating element or elements for a specific stimulation action on the upper limb.

Moreover, when in the same module there is simultaneously arranged a sensor and a stimulation element, it is worth to refer to this combination as a compact module that can be disposed along the wearable structure.

Alternatively, the stimulation elements of the stimulation means can be arranged within a stimulation module which, equal to module of sensors, is attachable in the proper location on the wearable structure.

Any of the modules are also attachable/detachable from the wearable structures.

Even further, within any of the described modules, there is a controller (control means) in order to manage the module itself. For example, in the module 31 shown in the figure 3, the module 31 is provided with a processor 311 for processing the sensing data and/or the stimulating data, communication means 312 to transmit or receive instructions, display element 313 to show a functioning status of the module (e.g., a pairing connection with other module) and power supply 314 for the power supply of the components of the module 31. Hence, the module 31 (and any module of the invention) is configured for autonomously functioning, being able to be paired with other modules, for example with the module 32, and with an external electronic portable device for further treatment of the data and/or for receive instructions data. This connection between modules generates a network or mesh of sensors or stimulation elements for sharing information that enables a comprehensive detection of the musculoskeletal condition of the upper limb (the forearm and the hand in this case) and also a comprehensive stimulation of the selected parts or portions of said upper limb.

Also, the wearable apparatus 10 comprises control means 50, i.e., actuating as central control of the apparatus, in data signal communication with the sensor or modules 31, 32, 33, 34, 35, wherein the control means 50 are configured to receive sensors data signals and/or stimulating data signals from the different sensors or stimulating elements placed along the wearable structure and/or from the modules and, based on said data signals, performing a diagnosis of the musculoskeletal condition (i.e., the bones, tendons, muscles, soft tissues) and the status of the joints, also, in this embodiment, applied force and muscle strength of the hand and/or the wrist of the user, movement of the fingers, etc., and, consequently, through the stimulation means, apply the suitable stimulation actions, according to task that must be performed for the wearable apparatus.

The control means 50 could be a microcontroller and/or microprocessor with its peripherical, suitable to be attachable or integrated in the wearable structure. Alternatively, an electric portable device 70, an external CPU or handheld device 70, like a smartphone or similar, is configured to receive the data signals from the different sensors, stimulating elements, and/or modules of the apparatus, and/or from the control means 50, and further for sending to the wearable apparatus 10 operational commands.

According to the above, the electric portable device 70 is provided with computer implemented program (software, app or the like), wherein the device 70 is configured to receive the data signals from the different sensors or stimulating elements and/or modules and processing said data through the computer implemented program and, from the results of processing, display data of the different sensors, stimulating elements or modules, and/or to display in real time the measurements of the different sensors, the musculoskeletal condition, and/or to display the action protocols for the prevention and/or recovery of a specific musculoskeletal disorders of the upper limb, and also to set the stimulation actions according to the purposes of the apparatus 10.

All the sensors, stimulation elements, and/or modules can be embedded or integrated by a process of transfer printing and conductive transferring, alongside hybrid and crossover electronics for electronic textiles, like inmold-technology.

### Exemplary embodiment 2: Lower limb (knee and leg)

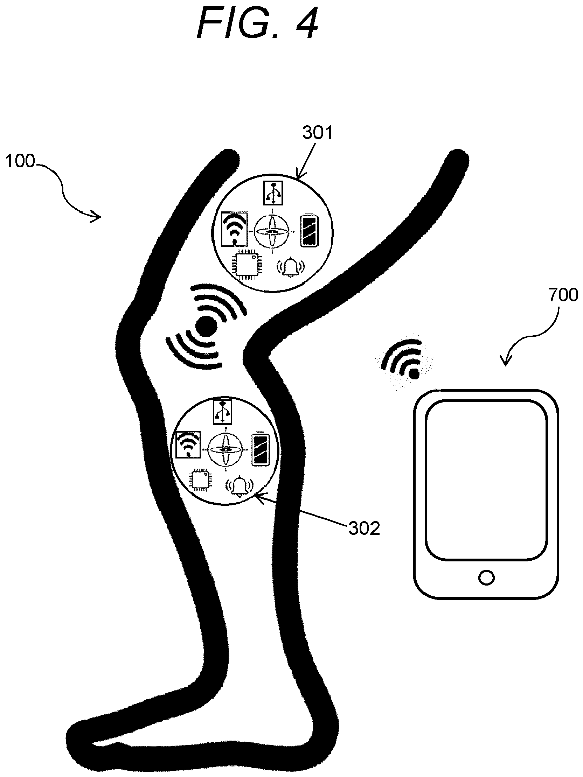

Taking into consideration the teachings of the aforementioned embodiments, in a second exemplary embodiment, shown in figure 4, the present invention discloses a wearable apparatus 100 intended to be attach on the lower limb to monitor a musculoskeletal condition of the knee and muscles of the lower limb and also to stimulate thereof.

As it is depicted in the figure 4, in the wearable structure (not shown) there is a compact module 301 above the knee and there is another compact module 302 below the knee, wherein above and below are referring to a standing position of a human being.

These modules 301 and 302 are wirelessly network connected between them and with an electric portable device 700. The latter being provided with a computer implemented program (software, app or the like), wherein the device 700 is configured to receive the data signals from the modules 301, 302 and processing said data through the computer implemented program and, from the results of processing, display data of the different sensors, stimulating elements or modules, and/or to display in real time the measurements of the different sensors, the musculoskeletal condition, and/or to display the action protocols for the prevention and/or recovery of a specific musculoskeletal disorders of the knee and/or the lower limb, and also to set the stimulation actions according to the purposes of the apparatus 100.

## Claims

1. A wearable apparatus to monitor a musculoskeletal condition of a joint, limb and muscles and also to stimulate thereof, comprising:
- a wearable structure configured to fit over the joint and/or the limb, and/or adjacent to the skin of the muscle;
- sensing means attachable to the wearable structure configured to detect the musculoskeletal condition of the joint and/or limb and/or muscles, to measure activity parameters of said joint and/or limb and/or muscles, and to generate sensing data signals related with the musculoskeletal condition and/or the activity parameters measured;
- stimulating means attachable to the wearable structure configured to provide a stimulation action to the joint and/or the limb and/or the muscles; and
- control means in data signal connection with the sensing means and with the stimulating means, wherein the control means are configured to monitor the musculoskeletal condition and the activity of the joint and/or limb and/or muscles based on the sensing data signals; and are further configured to manage the stimulating means based on the sensing data signal and/or on external data signals.

2. The wearable apparatus of the claim 1 wherein an artificial intelligence is implemented with the control means, configured to, based on a training from the monitoring of the musculoskeletal condition and/or the activity parameters measured and/or the stimulation action, generate action protocols for the prevention and/or recovery of a specific musculoskeletal disorders of joint and/or limb and/or muscles.

3. The wearable apparatus of the any of the previous claims wherein the sensing means comprising an automatic gain control configured to, based on the sensing data signals, calibrate the sensor means.

4. The wearable apparatus of any of the preceding claims wherein the sensing means comprises a movement sensor arrangement couplable to the wearable structure configured to measure the movement and/or the orientation of the joint and/or the limb.

5. The wearable apparatus of any of the preceding claims wherein the sensing means comprises a bio-feedback sensor arrangement couplable to the wearable structure configured to measure a bio-mark condition of the joint, limb and/or muscles.

6. The wearable apparatus of any of the preceding claims wherein the stimulating means comprises a stimulating sensor arrangement configured to stimulate the joint and/or the limb through temperature changes and/or vibration, and/or electrostimulation.

7. The wearable apparatus of any of the preceding claims further comprises displaying means operatively connected with the sensing means, and/or with the stimulating means and/or with the controller, being configured to visual feedback of the musculoskeletal condition and/or the activity of the joint and/or limb and/or muscles.

8. The wearable apparatus of any of the preceding claims wherein the control means are configured to transmit data and/or receive data to/from a portable electronic device, wherein the portable electronic device is configured to display data of the sensing means and/or the stimulating means.

9. A system to monitor a musculoskeletal condition of a joint, limb and muscles and also to stimulate thereof, comprising
- at least two wearable apparatus as the wearable apparatus of the claims 1 to 8, being placed each of those wearable apparatus in different joint, limb and muscles, wherein the control means of each wearable apparatus are operatively connected between them in order to share musculoskeletal conditions and/or sensing data signals, and/or stimulation actions.

10. The system of the previous claim wherein the control means of each wearable apparatus are wirelessly network connected.
